# EUROPEAN PATENT SPECIFICATION

(11) **EP 4 438 592 B1**
(45) Date of publication and mention of the grant of the patent: **11.02.2026**
(21) Application number: 22964190.7
(22) Date of filing: 21.11.2022
(51) Int. Cl.: C07C 247/14, C07C 271/24, C12P 13/02

(54) **EDOXABAN INTERMEDIATE AND PREPARATION METHOD THEREFOR**
EDOXABANZWISCHENPRODUKT UND HERSTELLUNGSVERFAHREN DAFÜR
INTERMÉDIAIRE D'EDOXABAN ET SON PROCÉDÉ DE PRÉPARATION

(30) Priority: 31.10.2022 CN 202211343010
(43) Date of publication of application: 02.10.2024
(73) Proprietor: Shanghai Bios Technology Co., Ltd., Shanghai 201499 (CN)
(72) Inventor: YU, Wansheng, Shanghai 201499 (CN); RUAN, Libo, Shanghai 201499 (CN); PENG, Qiujun, Shanghai 201499 (CN); ZENG, Yifei, Shanghai 201499 (CN)
(74) Representative: Bayramoglu et al.
(86) International application number: PCT/CN2022/133185
(87) International publication number: WO 2024/092892

(56) References cited:
- CN-A- 1 826 333
- CN-A- 101 263 110
- CN-A- 106 866 452
- KR-A- 20210 066 404
- RIOS-LOMBARDIA, NICOLAS ET AL.: "Chemoenzymatic one-pot synthesis in an aqueous medium: combination of metal-catalysed allylic alcohol isomerisation-asymmetric bioamination", CHEMICAL COMMUNICATIONS, vol. 51, no. 54, 26 May 2015 (2015-05-26), pages 10937 - 10940, XP055423389, ISSN: 1359-7345, DOI: 10.1039/C5CC03298A
- N. RICHTER: "ω-Transaminases for the amination of functionalised cyclic ketones", ORGANIC & BIOMOLECULAR CHEMISTRY, ROYAL SOCIETY OF CHEMISTRY, vol. 13, no. 33, 1 January 2015 (2015-01-01), pages 8843 - 8851, XP093167366, ISSN: 1477-0520, DOI: 10.1039/C5OB01204J

## Description

### TECHNICAL FIELD

The present invention belongs to the technical field of biocatalysis, in particular relates to a key edoxaban intermediate and an enzyme catalyzed preparation method therefor.

### BACKGROUND

Edoxaban p-toluenesulfonate monohydrate is developed by Daiichi Sankyo Inc., is a small molecule drug, and is a coagulation factor Xa inhibitor. At present, the highest stage of research and development of this drug is approved for marketing for the treatment of pulmonary embolism, venous thromboembolism, stroke, venous thrombosis and embolism. On April 22, 2011, Edoxaban tosylate was approved by the Japan Pharmaceutical and Medical Device Agency (PMDA) and sold by Daiichi Sankyo Inc. On January 8, 2015, Edoxaban tosylate was approved by the U.S. Food and Drug Administration (FDA), and sold by Daiichi Sankyo Inc. under a trade name of Savaysa^{®}. (NDA206316) On June 19, 2015, Edoxaban tosylate was approved by the European Medicines Agency (EMA), and sold by Daiichi Sankyo Europe Gmbh under a trade name of Lixiana^{®}. (EMEA/H/C/002629) On December 25, 2018, Edoxaban tosylate was approved by the National Medical Products Administration (NMPA) of China, and sold by Daiichi Sankyo Europe Gmbh under a trade name of LIXIANA^{®}.

Edoxaban has three chiral centers and a total of 8 isomers, but only one configuration has good activity, and a structure of Edoxaban is as follows:

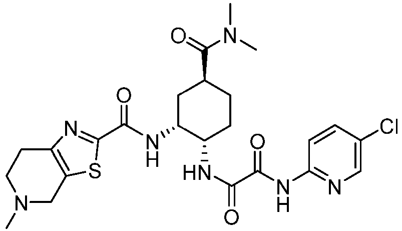

Edoxaban is developed by JP Daiichi Sankyo Inc, and a synthesis route thereof published in WO2008156159A1 is shown below. Racemic S-1 is resolved in the presence of a chiral amine S-2, followed by intramolecular nucleophilic attack under the action of an electrophilic brominating reagent to obtain a bridged ring compound S-4. The S-4 is subjected to aminolysis, followed by ring opening under the action of NH₄OH through a propylene oxide intermediate S-6 to obtain an amino alcohol compound S-7. Then, amino in the S-7 is protected with Boc, alcoholic hydroxyl in the S-7 is protected with Ms, and NaN₃ attack is to introduce a N₃ group to obtain a compound S-10. Subsequently, reduction by hydrogenation is performed to obtain amino, and the obtained compound and S-13 are subjected to condensation to obtain a compound S-14. The Boc protecting group of the compound S-14 is removed under acidic conditions, and then the obtained compound and carboxylic acid S-16 are subjected to condensation to obtain a final compound S-17, which forms a TsOH salt to obtain edoxaban. A synthetic route of this process is as follows:

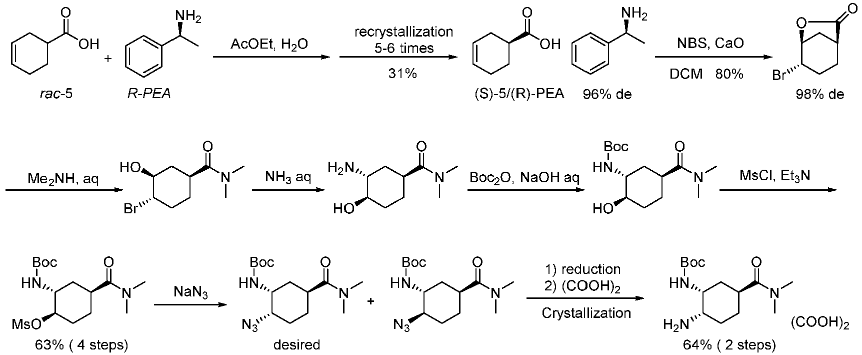

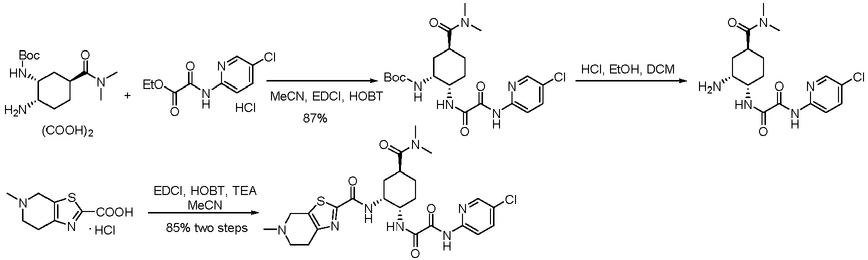

A difficulty in the synthesis of this compound lies in the preparation of chiral substrates such as the compound S-10.

A synthetic route disclosed in a patent CN101263110 by JP Daiichi Sankyo Inc: more than 10% of racemates appear in the substitution of sodium azide for mesylate in this route. A synthetic route of this process is as follows:

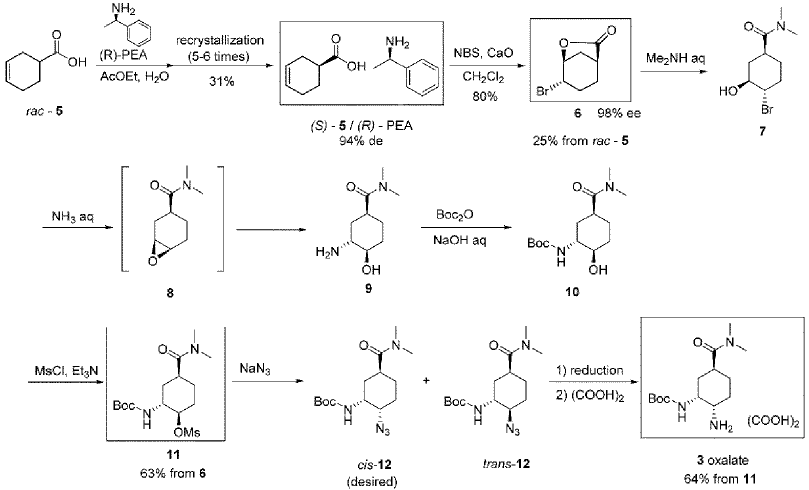

Daiichi Sankyo Inc. published the optimization of this process route in CN105008325A; DOI: 10.1021/acs.oprd.8b00413, a Burgess reagent is used, the racemization problem is avoided in the first generation route, and the yield is increased by 20%; however, the heat release during use of this reagent is intense, and the amplification effect is significant during amplification. A synthetic route of this process is as follows:

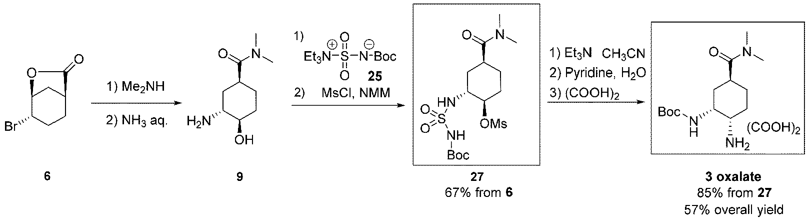

In CN106866452, a method for chemically constructing an amine chiral center is disclosed, which is difficult to reproduce, and a synthetic route of this process is as follows:

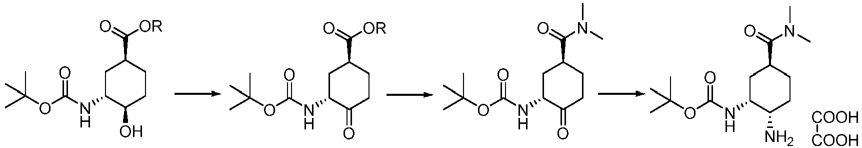

### SUMMARY

The technical problem to be solved by the present invention is to provide a method for preparing a key edoxaban intermediate by an enzyme catalyzed method.

In order to solve the above problem, the present invention adopts the following technical solutions:
an edoxaban intermediate has a general structural formula shown in a formula I or a formula II: 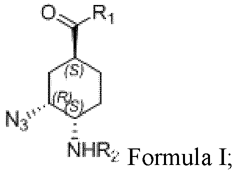 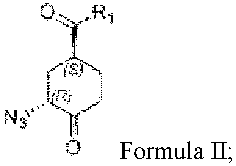
wherein R₁ is OH, alkoxy or a N,N-dialkyl group, and R₂ is hydrogen, alkoxycarbonyl or other amino protecting groups.

Preferably, in the general structural formula, R₁ is N,N-dimethyl or ethoxy, and R₂ is hydrogen, t-butoxycarbonyl, benzyloxycarbonyl, ethoxycarbonyl, benzyl or benzoyl.

The present invention further provides a preparation method for the edoxaban intermediate, including the steps of:
Step 1): subjecting a compound of a formula III to an oxidation reaction to obtain the compound of the formula II; 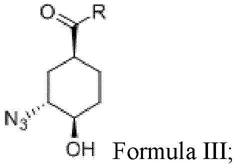 wherein a process route of the step 1) is as follows: 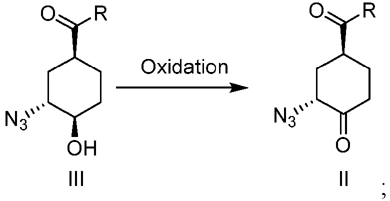 and
Step 2): mixing the compound of the formula II, an aminotransferase, an aminotransferase coenzyme, and a phosphate buffer solution for an enzyme catalyzed reaction or further amine derivatization to obtain the compound of the formula I.

A process route of the step 2) is as follows:

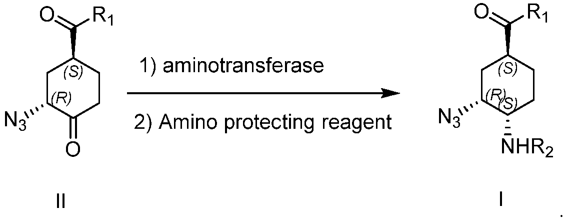

Preferably, an oxidizing agent in the step 1) is a John's reagent, a pyridinium chlorochromate (PCC) reagent or a 2,2,6,6-tetramethylpiperidinooxy (TEMPO) reagent, preferably the TEMPO reagent.

Preferably, the aminotransferase in the step 2) is selected from an aminotransferase enzyme bank of SyncoZymes (Shanghai) Co., Ltd.

Preferably, in a system of the step 2), the concentration of a substrate is 10-100 g/L; the aminotransferase participates in the reaction in the form of one or a combination of more of a wet bacterial body and a liquid enzyme; and when the aminotransferase is added in the form of the liquid enzyme, a mass proportion of the liquid enzyme is 1-20%, the reaction temperature is 0-30°C, a pH of the reaction is 6.5-7.0, and the reaction time is 12-30 h.

Preferably, in the step 2), the concentration of the compound of the formula II is 10-100 g/L, the concentration of the aminotransferase is 5-20 g/L, the concentration of the aminotransferase coenzyme is 1-10 mg/L, and the concentration of the phosphate buffer solution is 10-100 mM.

Preferably, after the amine derivatization is carried out to obtain an amine compound in the step 2), a characteristic reaction of a protecting group of an amine functional group is as follows: a protecting group reagent is directly added into a reaction solution obtained after the enzyme catalyzed reaction, and a reaction is carried out to obtain the compound of the formula I.

Preferably, the method further includes a post-treatment step: adding a filter aid into a reaction solution obtained after the reaction is finished in the step 2), and performing filtering, extracting, filtering, concentrating and crystallizing to obtain the compound of the formula I.

More preferably, the filter aid is diatomaceous earth.

The process route of the present invention is as follows:

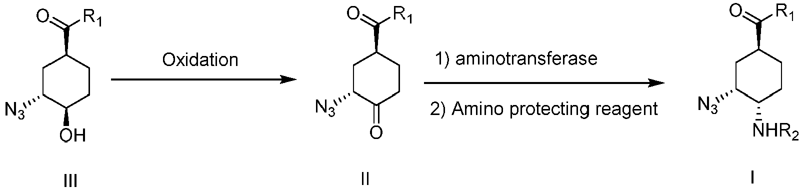

Further, the compound of the formula III can be prepared by the following method:

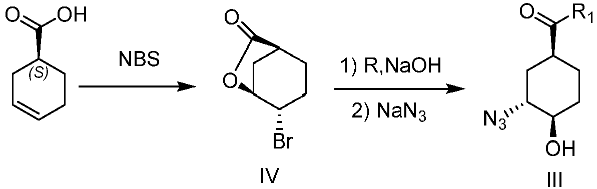

Compared with the prior art, the beneficial effects of the present invention are as follows:
the enzyme catalyzed preparation method for the compound of the formula I provided by the present invention can achieve high yield and chiral purity. A small amount of environment-friendly organic solvent is used, the reaction conditions are mild, the process operation is simple, and the method is suitable for industrial production.

In order to further confirm the key step described in the present invention, that is, a chiral configuration of an amine chiral center constructed by the aminotransferase, a compound of a formula 1-2 obtained in the present invention is derivatized as follows to obtain a compound of a formula V, and a single crystal X-ray diffraction pattern of the compound of the formula V is obtained through experiments, further confirming that an absolute configuration of a key intermediate obtained in the present invention is correct.

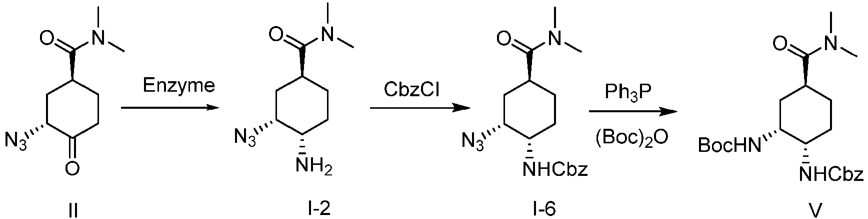

### BRIEF DESCRIPTION OF THE DRAWINGS

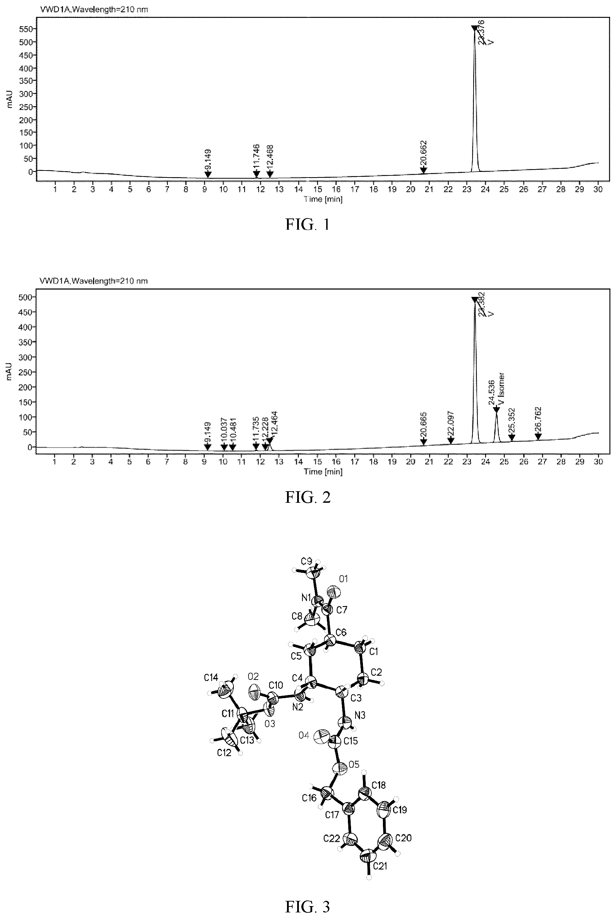

FIG. 1 is an HPLC spectrum of a compound of a formula V, tert-butyl ((1R,2S,5S)-2-(((benzyloxy)carbonyl)amino)-5-(dimethylcarbamoyl)cyclohexyl)-14-n itrocarboxylate;
FIG. 2 is an HPLC spectrum of the compound of the formula V, tert-butyl ((1R,2S,5S)-2-(((benzyloxy)carbonyl)amino)-5-(dimethylcarbamoyl)cyclohexyl)-14-n itrocarboxylate and a chiral isomer thereof; and
FIG. 3 is a single crystal X-ray diffraction pattern of the compound of the formula V, tert-butyl ((1R,2S,5S)-2-(((benzyloxy)carbonyl)amino)-5-(dimethylcarbamoyl)cyclohexyl)-14-n itrocarboxylate.

### DETAILED DESCRIPTION

In order to make the present invention more clearly understood, the present invention will be described in detail below by preferred examples with reference to the accompanying drawings.

### Example 1 Preparation of (15,4S,5S)-4-bromo-6-oxabicyclo[3.2.1]octan-7-one (IV)

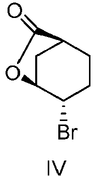

(S)-3-cyclohexenecarboxylic acid (126.2 g, 1.0 mol), 200 mL of water and potassium hydroxide (41 g, 1.05 mol) were sequentially added into a reaction flask; a reaction system was cooled to about -10°C under stirring, NBS (196 g, 1.1 mol) was slowly added, after the addition was finished, the temperature of the reaction system was maintained at -5°C to 5°C, and a reaction was continued to be carried out under stirring for 1 h; and after the reaction was complete by TLC monitoring, sodium sulfite (25.2 g, 0.2 mol) was added, a reaction was carried out under stirring for 0.5 h, and filtering was performed to give 198.8 g of a compound III, with a yield of 97%. H NMR data of the obtained product was as follows:
¹H NMR (400 MHz, Chloroform-d) δ 4.79 (d, J = 5.1 Hz, 1H), 4.39 (d, J = 4.7 Hz, 1H), 2.65 (d, J = 12.3 Hz, 2H), 2.46-2.35 (m, 1H), 2.35-2.27 (m, 1H), 2.13 (dd, J = 16.4, 5.4 Hz, 1H), 1.93 (dt, J = 12.9, 5.5, 2.2 Hz, 1H), 1.84 (dt, J = 12.1, 11.0, 4.9, 3.1 Hz, 1H).

A one-pot process for the preparation of a series of compounds III was represented by R being OCH₂CH₃ and N(CH₃)₂.

### Example 2 Preparation of ethyl (1S,3R,4R)-3-azido-4-hydroxycyclohexane-1-carboxylate (III-1)

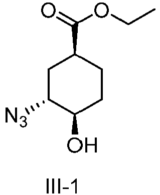

A compound IV (20.5 g, 0.1 mol) and 20 mL of ethanol were respectively added into a 100 mL three-necked flask, and cooled to 0-5°C, sodium ethoxide (10.2 g, 0.15 mol) was slowly added into the above reaction system, and a reaction was carried out under stirring while heat preservation at 0-5°C for 1-2 h; and 30 mL of water and sodium azide (9.7 g, 0.15 mol) were added into the above reaction system, the temperature was raised to 15-20°C, a reaction was carried out under stirring for 3 h, 50 mL of dichloromethane was added, liquid separation was performed, and a dichloromethane layer was concentrated under reduced pressure to remove dichloromethane to obtain a compound III-1 (18.7 g), with a yield of 88.4%. H NMR data of the obtained product was as follows:
¹H NMR (400 MHz, CDCl₃) δ 4.16 (dd, J = 7.2, 1.2 Hz, 2H), 3.52 (dt, J = 24.7, 8.5, 4.0 Hz, 2H), 2.72 (d, J = 4.8 Hz, 1H), 2.59-2.48 (m, 1H), 2.36 (dt, J = 14.0, 4.4 Hz, 1H), 2.14-2.05 (m, 1H), 1.95-1.85 (m, 1H), 1.63-1.46 (m, 3H), 1.26 (dt, J = 8.9, 5.1, 1.4 Hz, 3H).

### Example 3 Preparation of (1S,3R,4R)-3-azido-4-hydroxy-N,N-dimethylcyclohexane-1-carboxamide (III-2)

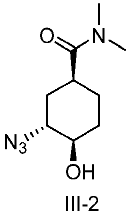

The compound IV (20.5 g, 0.1 mol) and 20 mL of acetonitrile were added into a 250 mL three-necked flask, and cooled to 0-5°C, a 40% aqueous dimethylamine solution (16.8 g, 0.15 mol) was added, and a reaction was carried out under stirring while heat preservation at 0-5°C for 10-11 h; dimethylamine and acetonitrile were distilled off under reduced pressure, 30 mL of water and sodium hydroxide (6 g, 0.15 mol) were added, and a reaction was carried out under stirring at 0-10°C for 5-6 h; and sodium azide (9.7 g, 0.15 mol) was weighed to be added into the system, the temperature was raised to 15-20°C, a reaction was carried out under stirring for 3-4 h, 50 mL of dichloromethane was added, liquid separation was performed, and a dichloromethane layer was concentrated under reduced pressure to remove dichloromethane to obtain a compound III-2 (19.1 g), with a yield of 90.1%. H NMR data of the obtained product was as follows:
¹H NMR (400 MHz, CDCl₃) δ 3.98 (dd, J = 10.0, 6.0 Hz, 1H), 3.64 (s, 1H), 3.43 (s, 1H), 3.03 (s, 3H), 2.91 (s, 3H), 2.88 (d, J = 3.9 Hz, 1H), 2.23-2.09 (m, 1H), 1.95-1.77 (m, 2H), 1.76-1.65 (m, 1H), 1.54 (dt, J = 16.4, 5.8 Hz, 2H); ¹³C NMR (101 MHz, CDCl₃) δ 174.93, 69.31, 62.25, 37.34, 35.70, 34.58, 29.01, 28.11, 23.48.

### Example 4 Preparation of ethyl (1S,3R)-3-azido-4-oxocyclohexane-1-carboxylate

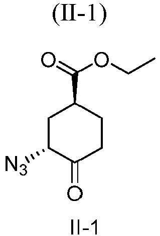

The compound III-1 (2.1 g, 0.01 mol), sodium bicarbonate (1.9 g, 0.02 mol), sodium bromide (0.1 g, 0.001 mol) and TEMPO (0.08 g, 4%, w/w) were added into a 100 mL three-necked flask, 15 mL of dichloromethane was added, the mixture was stirred to be dissolved, the temperature was reduced to -5°C to 0°C, and an aqueous solution of sodium hypochlorite (15.8 g, 0.015 mol) was weighed to be added dropwise into the reaction system slowly while keeping the temperature not more than 0°C; and after the addition dropwise was complete, stirring was continued to be performed for 0.5-1.0 h, the solution was left to stand, liquid separation was performed, and a dichloromethane layer was concentrated under reduced pressure to remove a solvent to give a compound II-1 (1.9 g), with a yield of 90.5%. H NMR data of the obtained product was as follows:
¹H NMR (400 MHz, CDCl₃) δ 4.16 (dd, J = 7.2, 1.2 Hz, 2H), 3.9 (d, J = 4.8 Hz, 1H), 2.72 (d, J = 4.8 Hz, 1H), 2.36 (dd, J = 14.0, 4.4 Hz, 1H), 2.14-2.05 (m, 1H), 1.95-1.85 (m, 1H), 1.63-1.46 (m, 3H), 1.26 (dd, J = 8.9, 5.1, 1.4 Hz, 3H); ¹³C NMR (125 MHz, CDCl₃) δ 209.75, 174.15, 60.85, 59.37, 39.24, 36.44, 34.75, 27.08, 14.26.

### Example 5 Preparation of (1S,3R)-3-azido-N,N-dimethyl-4-oxocyclohexane-1-carboxamide (II-2)

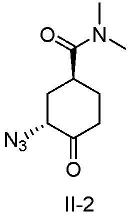

The compound III-2 (2.1 g, 0.01 mol), sodium bicarbonate (1.9 g, 0.02 mol), sodium bromide (0.1 g, 0.001 mol) and TEMPO (0.08 g, 4%, w/w) were added into a 100 mL three-necked flask, 15 mL of dichloromethane was added, the mixture was stirred to be dissolved, the temperature was reduced to -5°C to 0°C, and an aqueous solution of sodium hypochlorite (15.8 g, 0.015 mol) was weighed to be added dropwise into the reaction system slowly while keeping the temperature not more than 0°C; and after the addition dropwise was complete, stirring was continued to be performed for 0.5-1.0 h, the solution was left to stand, liquid separation was performed, and a dichloromethane layer was concentrated under reduced pressure to remove a solvent to give a compound II-2 (1.8 g), with a yield of 86.5%. H NMR data of the obtained product was as follows:
¹H NMR (400 MHz, CDCl₃) δ 4.68-4.56 (m, 1H), 3.17 (dd, J = 11.2, 6.7 Hz, 1H), 3.07 (s, 3H), 2.95 (s, 3H), 2.76- 2.63 (m, 1H), 2.49 (dd, J = 11.7, 4.9 Hz, 1H), 2.32 (dd, J = 13.4, 5.5 Hz, 1H), 2.14-2.02 (m, 1H), 2.02-1.91 (m, 1H), 1.91-1.79 (m, 1H); ¹³C NMR (101 MHz, CDCl₃) d 205.55, 173.40, 63.68, 37.21, 37.06, 35.27, 34.27, 33.94, 27.86.

### Example 6 Preparation of ethyl (1S,3R,4S)-4-amino-3-azidocyclohexane-1-carboxylate (I-1)

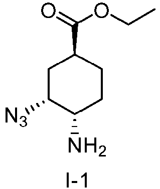

II-1 (5 g), PBS buffer (pH 7.0, 1 M, 50 mL), water (350 mL), PLP (2.6 mg, 5.3 mg/L), isopropylamine (100 mL, 50 g/L) and ATA101 (5.0 g) were added into a 500 mL reaction flask, and a reaction was carried out with mechanical stirring at 25-30°C for 25-30 h; and after completion of the reaction, filtration was performed with diatomaceous earth, and a mother liquor was extracted with dichloromethane to give a compound I-1 (4.3 g), with a yield of 86.5%.

### Example 7 Preparation of (1S,3R,4S)-4-amino-3-azido-N,N-dimethylcyclohexane-1-carboxamide (I-2)

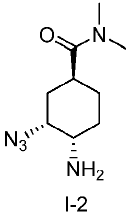

II-2 (5 g), PBS buffer (pH 7.0, 1 M, 50 mL), water (350 mL), PLP (2.6 mg, 5.3 mg/L), isopropylamine (50 mL, 100 g/L) and ATA101 (5.0 g) were added into a 500 mL reaction flask, and a reaction was carried out with mechanical stirring at 25-30°C for 25-30 h; and after completion of the reaction, filtration was performed with diatomaceous earth, and a mother liquor was extracted with dichloromethane to give a compound I-2. H NMR data of the obtained product was as follows:
¹H NMR (400 MHz, CDCl₃) δ 3.82 (d, J = 2.3 Hz, 1H), 2.99 (s, 3H), 2.85 (s, 3H), 2.79-2.63 (m, 2H), 1.92 (dd, J = 14.3, 2.7 Hz, 1H), 1.87-1.72 (m, 1H), 1.72-1.56 (m, 2H), 1.54-1.18 (m, 4H). ¹³C NMR (101 MHz, CDCl₃) δ 174.62, 64.49, 52.00, 37.08, 35.53, 33.66, 32.15, 30.05, 27.55.

### Example 8 Preparation of ethyl (1S,3R,4S)-3-azido-4-(tert-butoxycarbonyl)amino)cyclohexane-1-carboxylate

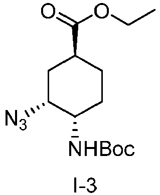

I-1 (2.1 g, 10 mmol), Boc₂O (2.6 g, 12 mmol), potassium carbonate (2.1 g, 15 mmol) and 50 mL of water were added into a 100 mL three-necked flask in one time, and heated to 40-50°C, a reaction was carried out under stirring for 3-4 h, so that a white solid was precipitated, and suction filtration was performed under reduced pressure to give 2.8 g of a compound I-3, with a yield of 90%.

### Example 9 Preparation of tert-butyl ((1S,2R,4S)-2-azido-4-(dimethylcarbamoyl)cyclohexyl)carbamate

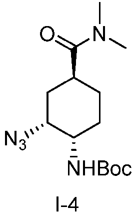

I-2 (2.1 g, 10 mmol), Boc₂O (2.6 g, 12 mmol), potassium carbonate (2.1 g, 15 mmol) and 50 mL of water were added into a 100 mL three-necked flask in one time, and heated to 40-50°C, a reaction was carried out under stirring for 3-4 h, so that a white solid was precipitated, and suction filtration was performed under reduced pressure to give 2.9 g of a compound I-4, with a yield of 94%. H NMR data of the obtained product was as follows:
¹H NMR (400 MHz, CDCl₃) δ 4.87 (d, J = 7.2 Hz, 1H), 4.03 (s, 1H), 3.54 (s, 1H), 2.95 (s, 3H), 2.82 (s, 3H), 2.70 (t, J = 11.1 Hz, 1H), 1.85 (dd, J = 31.3, 13.5 Hz, 2H), 1.66 (t, J = 13.1 Hz, 2H), 1.57 - 1.40 (m, 2H), 1.33 (s, 9H). ¹³C NMR (101 MHz, CDCl₃) δ 174.20, 154.89, 79.42, 61.16, 50.94, 37.00, 35.48, 33.51, 31.83, 28.24, 27.41, 26.40.

### Example 10 Preparation of methyl ((1S,2R,4S)-2-azido-4-(dimethylcarbamoyl)cyclohexyl)carbamate

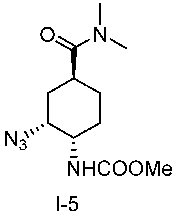

2-Methyltetrahydrofuran (80 mL) was added into I-2 (2.0 g), sodium bicarbonate (1.6 g, 2.0 eq) was added, methyl chloroformate (1.2 g, 1.3 eq) was added into the system with stirring at room temperature, stirring was performed while heat preservation for 2-3 h, after raw material conversion was complete by TLC detection, an insoluble substance was filtered off, a filter cake was washed with 2-methyltetrahydrofuran, a filtrate was subjected to liquid separation, an organic phase was concentrated, and recrystallization was performed with PE/EA to give I-5 as a white solid, with a yield of 84%. H NMR data of the obtained product was as follows:
¹H NMR (400 MHz, CDCl₃) δ 4.87 (d, J = 8.5 Hz, 1H), 4.13 (d, J = 4.2 Hz, 1H), 3.69 (s, 4H), 3.08 (s, 3H), 2.96 (s, 3H), 2.83 (d, J = 11.7, 3.6 Hz, 1H), 2.09-1.92 (m, 2H), 1.84-1.75 (m, 2H), 1.67-1.45 (m, 2H).

### Example 11 Preparation of benzyl ((1S,2R,4S)-2-azido-4-(dimethylcarbamoyl)cyclohexyl)carbamate

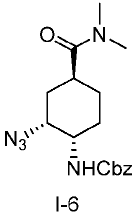

2-Methyltetrahydrofuran (80 mL) was added into I-2 (2.0 g), sodium bicarbonate (1.6 g, 2.0 eq) was added, benzyl chloroformate (2.1 g, 1.3 eq) was added into the system with stirring at room temperature, stirring was performed while heat preservation for 2-3 h, after raw material conversion was complete by TLC detection, an insoluble substance was filtered off, a filter cake was washed with 2-methyltetrahydrofuran, a filtrate was subjected to liquid separation, an organic phase was concentrated, and recrystallization was performed with PE/EA to give I-6 as a white solid, with a yield of 86%. H NMR data of the obtained product was as follows:
¹H NMR (400 MHz, CDCl₃) δ 7.41-7.30 (m, 5H), 5.12 (s, 2H), 4.95 (d, J = 8.5 Hz, 1H), 4.14 (s, 1H), 3.83-3.69 (m, 1H), 3.08 (s, 3H), 2.96 (s, 3H), 2.90-2.75 (m, 1H), 2.10-1.91 (m, 2H), 1.81 (d, J = 12.6 Hz, 2H), 1.69 (s, 1H), 1.58-1.46 (m, 1H).

### Example 12 Preparation of tert-butyl ((1R,2S,5S)-2-(((benzyloxy)carbonyl)amino)-5-(dimethylcarbamoyl)cyclohexyl)-14-n

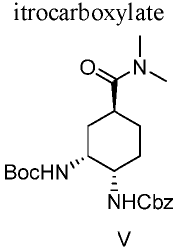

Triphenylphosphine (2.4 g, 3.0 eq), tetrahydrofuran (30 mL), and deionized water (7 mL) were added into I-6 (1.05 g), 1N HCl was added into the resulting reaction solution to adjust a pH to be 2-3 after completion of a reaction, concentration was performed to remove tetrahydrofuran, an aqueous phase was extracted with dichloromethane, an aqueous phase was retained, a pH of the aqueous phase was adjust to be 8-9 by adding sodium hydroxide, a Boc anhydride (1.0 g, 1.5 eq) was added, stirring was performed overnight, and suction filtration and water washing were performed to give a white solid V, with a yield of 85%. H NMR data of the obtained product was as follows:
¹H NMR (400 MHz, CDCl₃) δ 7.38-7.26 (m, 5H), 5.22 (s, 1H), 5.09 (t, J = 10.3 Hz, 2H), 4.12 (s, 1H), 3.70 (s, 1H), 3.02 (s, 3H), 2.92 (s, 3H), 2.62 (s, 1H), 1.99 (d, J = 37.6 Hz, 2H), 1.72 (d, J = 18.7 Hz, 5H), 1.43 (s, 9H).

## Claims

1. An edoxaban intermediate, **characterized by** having a general structural formula shown in a formula I or a formula II: 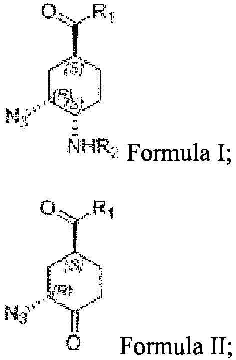 wherein R₁ is OH, alkoxy or a N,N-dialkyl group, and R₂ is hydrogen, alkoxycarbonyl or an amino protecting group.

2. The edoxaban intermediate according to claim 1, **characterized in that** in the general structural formula, R₁ is N,N-dimethyl or ethoxy, and R₂ is hydrogen, tert-butoxycarbonyl, benzyloxycarbonyl, ethoxycarbonyl, benzyl or benzoyl.

3. A preparation method for the edoxaban intermediate according to claim 1 or 2, **characterized by** comprising the steps of:
step 1): subjecting a compound of a formula III to an oxidation reaction to obtain the compound of the formula II; 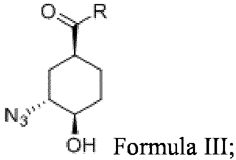 and
step 2): mixing the compound of the formula II, an aminotransferase, an aminotransferase coenzyme, and a phosphate buffer solution for an enzyme catalyzed reaction or further amine derivatization to obtain the compound of the formula I.

4. The preparation method for the edoxaban intermediate according to claim 3, **characterized in that** an oxidizing agent in step 1) is a John's reagent, a pyridinium chlorochromate (PCC) reagent or a 2,2,6,6-tetramethylpiperidinooxy (TEMPO) reagent.

5. The preparation method according to claim 3, **characterized in that** in a system of the step 2), the concentration of a substrate is 10-100 g/L; the aminotransferase participates in the reaction in the form of one or a combination of more of a wet bacterial body and a liquid enzyme; and when the aminotransferase is added in the form of the liquid enzyme, a mass proportion of the liquid enzyme is 1-20%, the reaction temperature is 0-30°C, a pH of the reaction is 6.5-7.0, and the reaction time is 12-30 h.

6. The preparation method according to claim 3, **characterized in that** in the step 2), the concentration of the compound of the formula II is 10-100 g/L, the concentration of the aminotransferase is 5-20 g/L, the concentration of the aminotransferase coenzyme is 1-10 mg/L, and the concentration of the phosphate buffer solution is 10-100 mM.

7. The preparation method according to claim 3, **characterized in that** after the amine derivatization is carried out to obtain an amine compound in the step 2), a characteristic reaction of a protecting group of an amine functional group is as follows: a protecting group reagent is directly added into a reaction solution obtained after the enzyme catalyzed reaction, and a reaction is carried out to obtain the compound of the formula I.

8. The preparation method according to claim 3, **characterized by** further comprising a post-treatment step: adding a filter aid into a reaction solution obtained after the reaction is finished in the step 2), and performing filtering, extracting, filtering, concentrating and crystallizing to obtain the compound of the formula I.

9. The preparation method according to claim 8, **characterized in that** the filter aid is diatomaceous earth.

## Patentansprüche

1. Edoxaban-Zwischenprodukt, **dadurch gekennzeichnet, dass** es eine allgemeine Strukturformel gemäß einer Formel I oder einer Formel II aufweist: 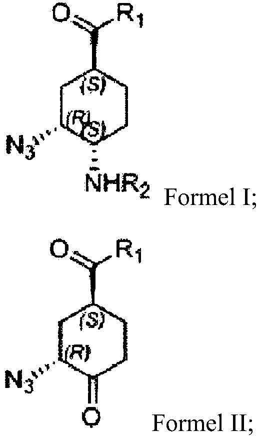 wobei R₁ OH, Alkoxy oder eine N, N-Dialkylgruppe ist und R₂ Wasserstoff, Alkoxycarbonyl oder eine Aminoschutzgruppe ist.

2. Edoxaban-Zwischenprodukt nach Anspruch 1, **dadurch gekennzeichnet, dass** in der allgemeinen Strukturformel R₁ N, N-Dimethyl oder Ethoxy ist und R₂ Wasserstoff, tert-Butoxycarbonyl, Benzyloxycarbonyl, Ethoxycarbonyl, Benzyl oder Benzoyl ist.

3. Herstellungsverfahren für Edoxaban-Zwischenprodukt nach Anspruch 1 oder 2, **dadurch gekennzeichnet, dass** es die Schritte umfasst:
Schritt 1): Unterziehen einer Verbindung der Formel III einer Oxidationsreaktion zum Erhalt der Verbindung der Formel II; 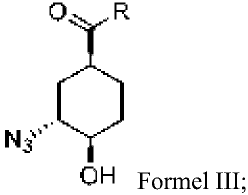 und
Schritt 2): Mischen der Verbindung der Formel II, einer Aminotransferase, eines Aminotransferase-Coenzyms und einer Phosphatpufferlösung für eine enzymkatalysierte Reaktion oder eine weitere Aminderivatisierung, um die Verbindung der Formel I zu erhalten.

4. Herstellungsverfahren für ein Edoxaban-Zwischenprodukt nach Anspruch 3, **dadurch gekennzeichnet, dass** ein Oxidationsmittel in Schritt 1) ein John's-Reagenz, ein Pyridinium chlorchromat-(PCC)-Reagenz oder ein 2,2,6,6-Tetramethylpiperidinooxy-(TEMPO)-Reagenz ist.

5. Herstellungsverfahren nach Anspruch 3, **dadurch gekennzeichnet, dass** in einem System des Schritts 2) die Konzentration eines Substrats 10-100 g/L beträgt; die Aminotransferase in der Reaktion in der Form eines oder einer Kombination von mehreren feuchten Bakterienzellen und eines flüssigen Enzyms teilnimmt; und wenn die Aminotransferase in der Form des flüssigen Enzyms zugegeben wird, ein Massenanteil des flüssigen Enzyms 1-20 % beträgt, die Reaktionstemperatur 0-30 °C beträgt, ein pH-Wert der Reaktion 6,5-7,0 beträgt und die Reaktionszeit 12-30 h beträgt.

6. Herstellungsverfahren nach Anspruch 3, **dadurch gekennzeichnet, dass** in Schritt 2) die Konzentration der Verbindung der Formel II 10-100 g/L beträgt, die Konzentration der Aminotransferase 5-20 g/L beträgt, die Konzentration des Aminotransferase-Coenzyms 1-10 mg/L beträgt und die Konzentration der Phosphatpufferlösung 10-100 mM beträgt.

7. Herstellungsverfahren nach Anspruch 3, **dadurch gekennzeichnet, dass** nach Durchführung der Aminderivatisierung zum Erhalt einer Aminverbindung in Schritt 2) eine charakteristische Reaktion einer Schutzgruppe einer Aminfunktionsgruppe wie folgt ist: ein Schutzgruppenreagenz wird direkt zu einer nach der enzymkatalysierten Reaktion erhaltenen Reaktionslösung zugegeben, und eine Reaktion wird durchgeführt, um die Verbindung der Formel I zu erhalten.

8. Herstellungsverfahren nach Anspruch 3, **dadurch gekennzeichnet, dass** es ferner einen Nachbehandlungsschritt umfasst: Zugeben eines Filterhilfsmittels zu einer nach Abschluss der Reaktion in Schritt 2) erhaltenen Reaktionslösung und Durchführen von Filtrieren, Extrahieren, Filtrieren, Konzentrieren und Kristallisieren, um die Verbindung der Formel I zu erhalten.

9. Herstellungsverfahren nach Anspruch 8, **dadurch gekennzeichnet, dass** das Filterhilfsmittel Kieselgur ist.

## Revendications

1. Intermédiaire d'édoxaban, **caractérisé en ce qu'**il présente une formule structurale générale représentée par une formule I ou une formule II : 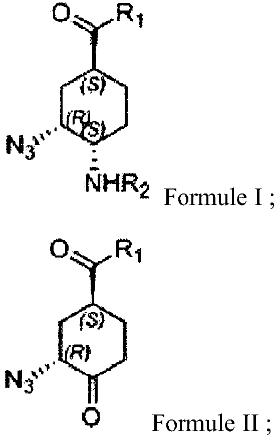 dans lequel R₁ est un groupe OH, un groupe alcoxy ou un groupe N,N-dialkyle, et R₂ est un atome d'hydrogène, un groupe alcoxycarbonyle ou un groupe protecteur amino.

2. Intermédiaire d'édoxaban selon la revendication 1, **caractérisé en ce que** dans la formule structurale générale, R₁ est un groupe N,N-diméthyl ou un groupe éthoxy, et R₂ est un atome d'hydrogène, un groupe tert-butoxycarbonyle, un groupe benzyloxycarbonyle, un groupe éthoxycarbonyle, un groupe benzyle ou un groupe benzoyle.

3. Procédé de préparation de l'intermédiaire d'édoxaban selon la revendication 1 ou 2, **caractérisé en ce qu'**il comprend les étapes suivantes :
étape 1) : soumission d'un composé de formule III à une réaction d'oxydation pour obtenir le composé de formule II ; 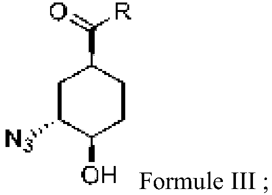 et
étape 2) : mélange du composé de formule II, d'une aminotransférase, d'une coenzyme aminotransférase et d'une solution tampon de phosphate pour une réaction catalysée par enzyme ou une dérivatisation amino supplémentaire pour obtenir le composé de formule I.

4. Procédé de préparation de l'intermédiaire d'édoxaban selon la revendication 3, **caractérisé en ce qu'**un agent oxydant de l'étape 1) est un réactif de John, un réactif de chlorochromate de pyridinium (PCC) ou un réactif 2,2,6,6-tétraméthylpipéridinooxy (TEMPO).

5. Procédé de préparation selon la revendication 3, **caractérisé en ce que**, dans le système de l'étape 2), la concentration d'un substrat est de 10-100 g/L ; l'aminotransférase participe à la réaction sous la forme d'un ou une combinaison de plusieurs corps bactériens humides et d'une enzyme liquide ; et lorsque l'aminotransférase est ajoutée sous forme d'enzyme liquide, la proportion massique de l'enzyme liquide est de 1-20 %, la température de réaction est de 0-30 °C, le pH de la réaction est de 6,5-7,0 et la durée de réaction est de 12-30h.

6. Procédé de préparation selon la revendication 3, **caractérisé en ce que**, à l'étape 2), la concentration du composé de formule II est de 10-100 g/L, la concentration de l'aminotransférase est de 5-20 g/L, la concentration de la coenzyme aminotransférase est de 1-10 mg/L et la concentration de la solution tampon de phosphate est de 10-100 mM.

7. Procédé de préparation selon la revendication 3, **caractérisé en ce que**, après la réalisation de la dérivatisation amino pour obtenir un composé aminé à l'étape 2), une réaction caractéristique d'un groupe protecteur d'un groupe fonctionnel amine est comme suit : un réactif de groupe protecteur est ajouté directement à une solution réactionnelle obtenue après la réaction catalysée par enzyme, et une réaction est réalisée pour obtenir le composé de formule I.

8. Procédé de préparation selon la revendication 3, **caractérisé en ce qu'**il comprend en outre une étape de post-traitement : ajout d'un adjuvant de filtration dans une solution réactionnelle obtenue à l'issue de la réaction à l'étape 2), et réalisation d'une filtration, d'une extraction, d'une filtration, d'une concentration et d'une cristallisation pour obtenir le composé de formule I.

9. Procédé de préparation selon la revendication 8, **caractérisé en ce que** l'adjuvant de filtration est de la terre de diatomée.
